(19) Europäisches Patentamt
European Patent Office
Office européen des brevets

(11) Veröffentlichungsnummer: **0 019 719**
**B1**

(12) **EUROPÄISCHE PATENTSCHRIFT**

(45) Veröffentlichungstag der Patentschrift: **21.07.82**

(21) Anmeldenummer: **80102209.6**

(22) Anmeldetag: **24.04.80**

(51) Int. Cl.³: **C 07 C 25/24**, C 07 C 49/327, C 07 C 49/355, C 07 C 49/567, C 07 C 61/40, C 07 C 69/743, C 07 C 69/757, C 07 C121/48, C 07 F 9/40

(54) **Verfahren zur Herstellung von Alkenen, neue Zwischenprodukte zu ihrer Herstellung und Verfahren zu deren Herstellung.**

(30) Priorität: **02.05.79 DE 2917620**
**21.07.79 DE 2929636**

(43) Veröffentlichungstag der Anmeldung: **10.12.80 (Patentblatt 80/25)**

(45) Bekanntmachung des Hinweises auf die Patenterteilung: **21.07.82 Patentblatt 82/29**

(84) Benannte Vertragsstaaten: **BE CH DE FR GB IT LI NL**

(56) Entgegenhaltungen:
**DE - A - 2 738 150**

**CHEMISCHE BERICHTE, Band 103, 1970 Weinheim/Bergstrasse L. HORNER et al. « Notiz über reduktive Umwandlung von Carbonsäuren in ihre Aldehyde » Seiten 2 984 bis 2 986**

**TETRAHEDRON LETTERS, Nr. 23, 1976 London T. MATSUO et al. « Synthesis of Optically Active Trans-Chrysanthemic Acid from Optically Active Pantolactone » Seiten 1 979 bis 1 982**

(73) Patentinhaber: **BAYER AG**
**Zentralbereich Patente, Marken und Lizenzen**
**D-5090 Leverkusen 1, Bayerwerk (DE)**

(72) Erfinder: **Hoffmann, Hellmut, Prof. Dr.**
**Tersteegenweg 17**
**D-5600 Wuppertal 1 (DE)**
Erfinder: **Maurer, Fritz, Dr.**
**Roeberstrasse 8**
**D-5600 Wuppertal 1 (DE)**
Erfinder: **Priesnitz, Uwe, Dr.**
**Heinrich-Heine-Strasse 42**
**D-4750 Unna-Massen (DE)**
Erfinder: **Riebel, Hans-Jochem, Dr.**
**In der Beek 92**
**D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 019 719 B1

Imprimerie Jouve, 18, rue St-Denis, 75001 Paris, France

Verfahren zur Herstellung von Alkenen, neue Zwischenprodukte zu ihrer Herstellung und Verfahren zu deren Herstellung

Die Erfindung betrifft ein neues Verfahren zur Herstellung von bekannten und neuen Alkenen, neue Zwischenprodukte zu ihrer Herstellung und Verfahren zur Herstellung der Zwischenprodukte.

Es ist bekannt, daß man Alkene, wie z.B. den als Zwischenprodukt für insektizid wirksame Pyrethroide verwendbaren 3-(2-Methyl-2-methoxy-carbonyl-vinyl)-2,2-dimethyl-cyclopropan-1-carbonsäure-tert.-butyl-ester, erhält, wenn man Aldehyde oder Ketone, wie z.B. 3-Formyl-2,2-dimethyl-cyclopropan-1-carbonsäure-tert.-butyl-ester, mit Phosphor-Yliden (Phosphor-Ylenen), wie z.B. (1-Methoxy-carbonyl-äthyliden)-phosphonsäure-diäthylester, umsetzt (vergleiche Organic Reactions, Vol. *25* (1977), S. 73-253, insbesondere S. 85 und S. 121 — und dort zitierte Literatur).

Weiter ist — als Spezialfall der vorstehend angegebenen Synthesemethode — bekannt, daß man bestimmte 3-(2-Chlor-2-phenyl-vinyl)-2,2-dimethyl-cyclopropan-1-carbonsäureester, wie z.B. 3-(2-Chlor-2-phenyl-vinyl)-2,2-dimethyl-cyclopropan-1-carbonsäure-äthylester erhält, wenn man entsprechende Benzyl-phosphonsäureester mit einer starken Base, wie z.B. Butyl-lithium, behandelt und die intermediär gebildeten Ylide mit Tetrachlorkohlenstoff und dann mit 3-Formyl-2,2-dimethyl-cyclopropan-1-carbonsäureestern umsetzt. (vergleiche DE-OS 2 738 150).

Die oben allgemein dargestellte Carbonyl-Olefinierungs-Reaktion besitzt zwar eine sehr große Anwendungsbreite, ist aber beispielsweise dann für industrielle Belange nicht mehr interessant, wenn die als Ausgangsstoffe einzusetzenden Aldehyde (oder Ketone) nur mit sehr hohem Aufwand hergestellt werden können. So erhält man nach dem Stand der Technik die als Pyrethroid-Zwischenprodukte verwendbaren 3-Formyl-2,2-dimethyl-cyclopropan-1-carbonsäureester durch Ozonolyse von 3-Alkenyl-2,2-dimethyl-cyclopropan-1-carbonsäureestern, z.B. Chrysanthemumsäureäthylester, welche ihrerseits aus natürlich vorkommenden Pyrethroiden herzustellen sind (vergleiche US-PS 3 679 667). Eine technisch einfach durchführbare Herstellungsmethode für 3-Formyl-2,2-dimethylcyclopropan-1-carbonsäureester ist nicht bekannt.

Einen hohen Aufwand würde die technische Durchführung der oben erläuterten, bekannten Methode zur Synthese von 3-(2-Chlor-2-phenyl-vinyl)-2,2-dimethyl-cyclopropan-1-carbonsäureestern erfordern :

Die zur Ylidbildung aus Benzylphosphonsäureestern benötigten starken Basen, wie z.B. Butyllithium, sind feuchtigkeits- und luftempfindlich ; daher wird die Reaktion in einer Inertgasatmosphäre, beispielsweise unter Stickstoff oder Argon und unter Verwendung eines inerten, sorgfältig getrockneten Verdünnungsmittels durchgeführt. Da die Umsetzung bei tiefer Temperatur, etwa bei − 70 °C zu erfolgen hat, muß das Reaktionsgemisch auch intensiv gekühlt werden. Die Aufarbeitung zur Isolierung der gewünschten Produkte, welche man als Isomerengemische erhält, ist ebenfalls aufwendig ; sie umfaßt mehrere Extraktionen und Lösungsmitteldestillationen sowie einen chromatographischen Trennungsprozeß. Aus diesen Gründen ist die bekannte Synthesemethode für eine Herstellung von 3-(2-Chlor-2-phenyl-vinyl)-2,2-dimethyl-cyclopropan-1-carbon-säureestern im industriellen Maßstab wenig geeignet.

Die Erfindung betrifft die Gegenstände (1) bis (3), nämlich :

1. ein Verfahren zur Herstellung von Alkenen der Formel (I)

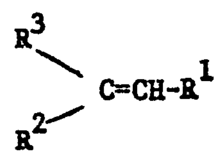

(I)

in welcher

$R^1$ für einen gegebenenfalls substituierten Alkyl-, Alkenyl-, Alkinyl-, Cycloalkyl-, Cycloalkenyl-, Aralkyl-, Aralkenyl- oder Aryl-Rest oder für einen gegebenenfalls substituierten heterocyclischen Rest steht,

$R^2$ für Wasserstoff oder Halogen steht und

$R^3$ für Halogen, Cyano, für einen gegebenenfalls substituierten Alkyl-, Alkenyl-, Alkinyl-, Cycloalkyl-, Aralkenyl-, Aryl-, Alkylcarbonyl-, Arylcarbonyl-, Alkoxycarbonyl- oder Aminocarbonyl-Rest oder für einen gegebenenfalls substituierten heterocyclischen Rest steht,

dadurch gekennzeichnet, daß man α-Hydroxy-phosphonsäureester der Formel II

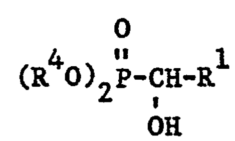

(II)

in welcher

$R^1$ die oben angegebene Bedeutung hat und

$R^4$ für Alkyl oder Phenyl steht oder die beiden Reste $R^4$ zusammen für geradkettiges oder

verzweigtes Alkandiyl (Alkylen) stehen, mit phosphorhaltigen Olefinierungsmitteln der Formel III

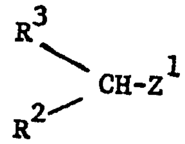

(III)

in welcher

$R^2$ und $R^3$ die oben angegebenen Bedeutungen haben und

$Z^1$ für einen phosphorhaltigen Rest

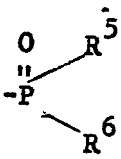 oder $-P(R^7)_3^{\oplus}$ $X^{\ominus}$

steht, wobei

$R^5$ und $R^6$ gleich oder verschieden sind und einzeln für Alkyl, Phenyl, Alkoxy oder Phenoxy oder zusammen für Alkandioxy stehen,

$R^7$ für Alkyl oder Phenyl steht und

X für Halogen steht,

in Gegenwart von Basen, gegebenenfalls in Gegenwart von Phasentransfer-Katalysatoren und gegebenenfalls unter Verwendung von Verdünnungsmitteln bei Temperaturen zwischen − 70 und + 150 °C umsetzt ;

2. neue α-Hydroxy-phosphonsäureester der Formel II a

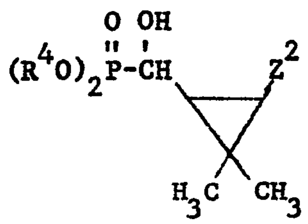

(IIa)

in welcher

$R^4$ für Alkyl oder Phenyl steht oder die beiden Reste $R^4$ zusammen für geradkettiges oder verzweigtes Alkandiyl (Alkylen) stehen und

$Z^2$ für Cyano, Carbamoyl, Acetyl oder $C_1$-$C_4$-Alkoxy-carbonyl steht ;

sowie

3. ein Verfahren zur Herstellung von α-Hydroxy-phosphonsäureestern der Formel IIa (oben), dadurch gekennzeichnet, daß man α-Oxo-phosphonsäureester der Formel IV

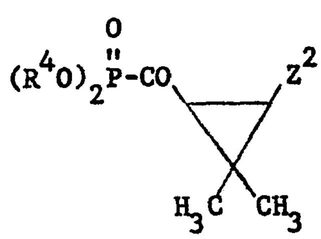

(IV)

in welcher

$R^4$ und $Z^2$ die unter (2) angegebenen Bedeutungen haben,

mit Hydridkomplexen der Formel V

$$M(M'H_4) \quad (V)$$

in welcher

M für Lithium, Natrium oder Kalium steht und

M' für Bor oder Aluminium steht,
gegebenenfalls in Gegenwart eines Puffermittels und gegebenenfalls unter Verwendung eines Verdünnungsmittels bei Temperaturen zwischen − 20 und + 50 °C umsetzt.

Die α-Oxo-phosphonsäureester der Formel IV

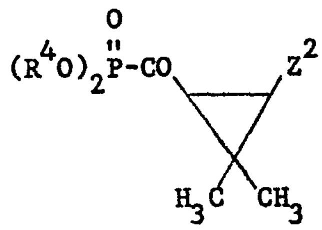

(IV)

in welcher

$R^4$ für Alkyl oder Phenyl steht oder die beiden Reste $R^4$ zusammen für geradkettiges oder verzweigtes Alkandiyl (Alkylen) stehen und

$Z^2$ für Cyano, Carbamoyl, Acetyl oder $C_1$-$C_4$-Alkoxycarbonyl steht,
werden erhalten, indem man Carbonsäurechloride der Formel VI

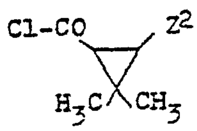

(VI)

in welcher

$Z^2$ die vorstehend angegebene Bedeutung hat,
mit Phosphorigsäureestern der Formel VII

$$R^8O—P(OR^4)_2 \qquad (VII)$$

in welcher

$R^4$ die vorstehend angegebene Bedeutung hat und

$R^8$ für Methyl oder Ethyl steht,
bei Temperaturen zwischen − 20 und + 150 °C, vorzugsweise zwischen 0 und 120 °C (vgl. J. Am. Chem. Soc. *86* (1964), 3862-3866 und Methoden der Organischen Chemie (Houben-Weyl-Müller 4. Aufl. Band *12/1,* S. 453, Georg Thieme-Verlag, Stuttgart 1963), umsetzt. Zur Isolierung und Reinigung der Produkte wird gegebenenfalls unter vermindertem Druck destilliert.

Die Carbonsäurechloride der Formel VI

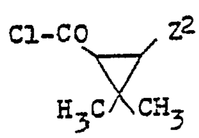

(VI)

in welcher

$Z^2$ für Cyano, Carbamoyl, Acetyl oder $C_1$-$C_4$-Alkoxycarbonyl steht,
werden erhalten, indem man Carbonsäuren der Formel VIII

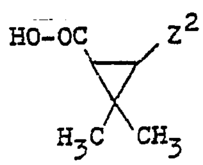

(VIII)

in welcher

$Z^2$ die vorstehend angegebene Bedeutung hat,
mit einem Chlorierungsmittel bei Temperaturen zwischen 20 und 80 °C umsetzt.

Gegenstand der vorliegenden Erfindung sind ferner

Die Styryl-cyclopropan-derivate der Formel XI sind neu

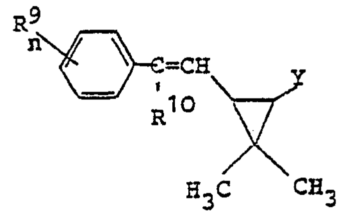

(XI)

in welcher

$R^9$ für Halogen oder für gegebenenfalls halogen-substituierte Reste Alkyl, Alkoxy, Alkylthio oder Alkylendioxy sowie für Cyano oder Nitro steht,

n für Null bis 5 steht,

$R^{10}$ für Wasserstoff oder Halogen steht und

Y für Acetyl, Cyano oder Carbamoyl steht.

Überraschenderweise können nach dem Verfahren gemäß Anspruch 1, welches einfach und kostengünstig durchzuführen ist und für welches ohne großen Aufwand herzustellende Ausgangsverbindungen einzusetzen sind, Alkene der Formel (I) in guten Ausbeuten und in hoher Reinheit erhalten werden.

Vorteile des erfindungsgemäßen Verfahrens liegen weiter beispielsweise in der Möglichkeit, die Umsetzung bei Raumtemperatur oder zumindest bei nicht weit davon abweichenden Temperaturen durchzuführen, billige Basen wie Alkali-hydroxide oder -alkoholate zu verwenden und wasserhaltige Lösungsmittel einzusetzen.

Ein besonderer Vorteil des erfindungsgemäßen Verfahrens besteht darin, daß die Herstellung von Aldehyden, welche im allgemeinen als Reaktionskomponenten bei der Herstellung von Alkenen durch Umsetzung von Carbonylverbindungen mit phosphorhaltigen Olefinierungsmitteln eingesetzt werden, vermieden werden kann.

Verwendet man als Ausgangsstoffe beispielsweise α-(3-Methoxy-carbonyl-2,2-dimethyl-cycloprop-1-yl)-α-hydroxy-methan-phosphonsäure-dimethylester und α-Chlorbenzylphosphonsäure-diethylester, so kann die erfindungsgemäße Reaktion dieser Verbindungen durch folgendes Formelschema skizziert werden :

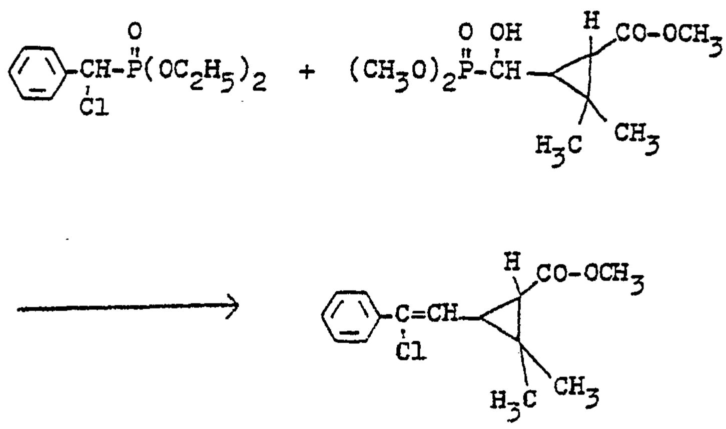

Die als Ausgangsstoffe zu verwendenden α-Hydroxyphosphonsäureester sind durch Formel (II) definiert. Vorzugsweise stehen darin

$R^4$ für $C_1$-$C_4$-Alkyl oder für Phenyl, oder beide Reste $R^4$ stehen gemeinsam für 2,2-Dimethyl-propan-1,3-diyl und

$R^1$ für gegebenenfalls halogen-substituiertes $C_1$-$C_4$-Alkyl, für gegebenenfalls durch Halogen, Cyano, Carbamoyl, $C_1$-$C_4$-Alkyl, $C_1$-$C_4$-Alkyl-carbonyl, $C_1$-$C_4$-Alkoxy-carbonyl und/oder Phenoxybenzyloxycarbonyl (welches gegebenenfalls durch Fluor, Cyano und/oder Ethinyl substituiert ist) substituiertes $C_3$-$C_6$-Cycloalkyl, für gegebenenfalls durch Chlor substituiertes Benzyl oder Phenylethyl, oder für gegebenenfalls durch Halogen, $C_1$-$C_4$-Alkyl und/oder $C_1$-$C_4$-Alkoxy substituiertes Phenyl.

Bei einer besonders bevorzugten Gruppe von (neuen) Ausgangsverbindungen der Formel (II) steht in dieser Formel

$R^1$ für den Rest

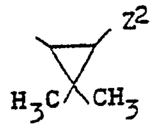

worin $Z^2$ für Cyano, Carbamoyl, Acetyl oder $C_1$-$C_4$-Alkoxycarbonyl steht und

$R^4$ für Methyl oder Ethyl.

Als Beispiele für Verbindungen der Formel (II) seien genannt:

α-Hydroxy-3-methyl-benzyl-,
α-Hydroxy-4-methyl-benzyl-,
α-Hydroxy-3,4-dimethyl-benzyl-,
α-Hydroxy-4-fluor-benzyl-,
α-Hydroxy-3-chlor-benzyl-,
α-Hydroxy-4-chlor-benzyl-,
α-Hydroxy-3,4-dichlor-benzyl-,
α-Hydroxy-3-brom-benzyl-,
α-Hydroxy-4-brom-benzyl-,
α-Hydroxy-4-methoxy-benzyl- und
α-Hydroxy-3,4-dimethoxy-benzyl-phosphonsäure-dimethylester und -diethylester;

α-Hydroxy-α-(3-methoxycarbonyl-2,2-dimethyl-cycloprop-1-yl)-methanphosphonsäure-dimethylester und -diethylester, α-Hydroxy-α-(3-äthoxycarbonyl-2,2-dimethyl-cycloprop-1-yl)-methanphosphonsäure-dimethyl-ester und -diethylester, α-Hydroxy-α-(3-acetyl-2,2-dimethyl-cycloprop-1-yl)-methanphosphonsäuredimethylester und -diethylester und α-Hydroxy-α-(3-cyano-2,2-dimethyl-cycloprop-1-yl)-methanphosphonsäure-dimethylester und -diethylester.

Die Ausgangsverbindungen der Formel (II) sind zum Teil noch nicht in der Literatur beschrieben, können aber nach im Prinzip bekannten Verfahren hergestellt werden. So erhält man die neuen α-Hydroxy-methan-phosphonsäureester der Formel (IIa), indem man die entsprechenden Oxoverbindungen der Formel IV (oben) mit einem Reduktionsmittel der Formel V (oben) wie z.B. Natriumtetrahydridoborat (Natriumboranat), gegebenenfalls unter Verwendung eines Verdünnungsmittels wie z.B. Wasser oder wässriges Methanol, bei Temperaturen zwischen − 20 und + 50 °C, vorzugsweise zwischen − 10 und + 30 °C, umsetzt und durch Zugabe eines Puffermittels wie z.B. Natriumhydrogenphosphat den pH-Wert zwischen 5 und 8 hält (vergleiche Chem. Ber. *103* (1970), 2984-2986). Zur Aufarbeitung und Isolierung der Produkte extrahiert man mit einem mit Wasser nicht mischbaren Lösungsmittel wie z.B. Methylenchlorid, trocknet die organische Phase, filtriert und destilliert das Lösungsmittel unter vermindertem Druck ab.

Ein Alternativweg zur Herstellung eines Teils der Verbindungen der Formel (II) — siehe Formel (IIIa) — ist weiter unten beschrieben.

Die Cyclopropancarbonsäurechloride der Formel (VI) erhält man gemäß nachstehendem Reaktionsschema aus bekannten Cyclopropan-carbonsäureestern (vergleiche J. Am. Chem. Soc. *89* (1967), 3912-3914; J. Org. Chem. *32* (1967), 3351-3355; Tetrahedron Lett. *1978*, 1847-1850; Bull. Soc. Chim. Belg. *87* (1978), 721-732) nach an sich bekannten Methoden, indem man zunächst durch Hydrolyse, beispielsweise durch Umsetzung mit wässrig-alkoholischer Kalilauge bei Temperaturen zwischen 20 und 100 °C und anschließendes Ansäuern die entsprechenden Cyclopropancarbonsäuren herstellt und diese mit Halogenierungsmitteln, wie z.B. Thionylchlorid, bei Temperaturen zwischen 20 und 80 °C umsetzt.

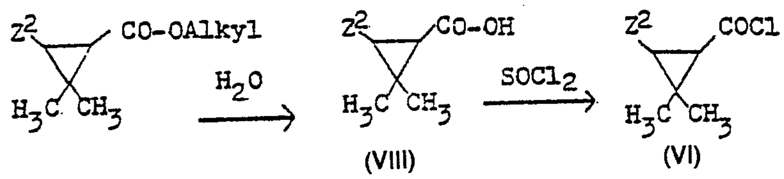


(VIII) (VI)

Die weiter als Ausgangsstoffe zu verwendenden phosphorhaltigen Olefinierungsmittel sind durch Formel (III) definiert. Vorzugsweise stehen darin

$R^2$ für Wasserstoff, Chlor oder Brom,

$R^3$ für Phenyl, welches gegebenenfalls durch Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_2$-(Halo)Alkylendioxy, $C_1$-$C_4$-Alkylthio und/oder $C_1$-$C_2$-Halogenalkylthio substituiert ist, und

$Z^1$ steht vorzugsweise für den Rest

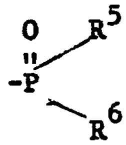

worin $R^5$ und $R^6$ gleich oder verschieden sind und einzeln für $C_1$-$C_4$-Alkoxy, Phenoxy oder zusammen für 2,2-Dimethyl-propan-1,3-dioxy stehen.

Als Beispiele für Verbindungen der Formel (III) seien genannt : Benzylphosphonsäure-dimethylester und -diethylester, α-Chlor-benzylphosphonsäure-dimethylester und -diethylester, α-Brom-benzylphosphonsäure-dimethylester und -diethylester, 4-Fluor-, 3-Chlor-, 4-Chlor-, 3,4-Dichlor-, 3-Brom-, 4-Brom-, 3-Methyl-, 4-Methyl-, 3,4-Dimethyl-, 4-Methoxy- und 3,4-Dimethoxy-benzylphosphonsäuredimethylester und -diethylester ;

4-Fluor-, 4-Chlor-, 3-Chlor-, 3,4-Dichlor-, 3-Brom-, 4-Brom-, 3-Methyl-, 4-Methyl-, 3,4-Dimethyl-, 4-Methoxy- und 3,4-Dimethoxy-α-chlor-benzylphosphonsäuredimethylester und -diethylester ;

4-Fluor-, 3-Chlor-, 4-Chlor-, 3,4-Dichlor-, 3-Brom-, 4-Brom-, 3-Methyl-, 4-Methyl-, 3,4-Dimethyl-, 4-Methoxy- und 3,4-Dimethoxy-α-brom-benzylphosphonsäure-dimethylester und -diethylester.

Die phosphorhaltigen Olefinierungsmittel der Formel (III) können nach im Prinzip bekannten Verfahren hergestellt werden.

Unter Formel (III) fallende, in α-Stellung unsubstituierte Benzyphosphonsäureester erhält man durch Umsetzung von Benzylhalogeniden mit Estern der phosphorigen Säure (vergleiche Methoden der organischen Chemie (Houben-Weyl-Müller), 4. Aufl. Band *12/1*, S. 433-453, Georg-Thieme-Verlag, Stuttgart 1963).

Unter Formel (III) fallende α-Chlor- und α-Brom-benzyl-phosphonsäureester erhält man durch Umsetzung von α-Hydroxy-benzylphosphonsäureestern der Formel IIIa

$$R^3-\underset{OH}{CH}-\overset{O}{\overset{\|}{P}}(OR^4)_2 \qquad \text{(IIIa)}$$

in welcher

$R^4$ die oben angegebene Bedeutung hat und

$R^3$ für gegebenenfalls substituiertes Phenyl steht,

mit einem Chlorierungsmittel, wie z.B. Thionylchlorid, bzw. mit einem Bromierungsmittel, wie z.B. Dibromtriphenylphosphoran, gegebenenfalls in Gegenwart eines Säureakzeptors, wie z.B. Pyridin, und gegebenenfalls unter Verwendung eines Verdünnungsmittels, wie z.B. Methylenchlorid oder Ethylenchlorid, bei Temperaturen zwischen 10 und 100 °C bzw. zwischen − 50 und + 50 °C (vergleiche J. Am. Chem. Soc. *87* (1965), 2777-2778 ; Chimia *28* (1974), 656-657).

Die α-Hydroxy-benzylphosphonsäureester der Formel (IIIa) erhält man nach dem oben angegebenen Weg zur Herstellung von Verbindungen der Formel (II) oder durch Umsetzung von Benzaldehyden der Formel IX

$$R^3—CHO \qquad \text{(IX)}$$

in welcher

$R^3$ die oben angegebene Bedeutung hat,

mit Phosphorigsäureestern der Formel X

$$H-\overset{O}{\overset{\|}{P}}(OR^4)_2 \qquad \text{(X)}$$

in welcher

$R^4$ die oben angegebene Bedeutung hat,

gegebenenfalls in Gegenwart eines Katalysators, wie z.B. Triethylamin, bei Temperaturen zwischen 0 und 150 °C, vorzugsweise zwischen 20 und 100 °C (vergleiche Methoden der organischen chemie (Houben-Weyl-Müller), 4. Aufl. Band *12/1*, S. 475-483, Georg-Thieme-Verlag, Stuttgart 1963).

Die Aldehyde der Formel IX und die Phosphorigsäureester der Formel (X) sind weitgehend bekannt.

Das erfindungsgemäße Verfahren zur Herstellung von Alkenen der Formel (I) wird vorzugsweise unter Verwendung geeigneter Lösungs- oder Verdünnungsmittel durchgeführt. Als solche kommen neben Wasser praktisch alle inerten organischen Solventien infrage. Hierzu gehören insbesondere aliphatische und aromatische, gegebenenfalls chlorierte Kohlenwasserstoffe, wie Pentan, Hexan, Heptan, Cyclohexan, Benzol, Toluol, Xylol, Methylenchlorid, Ethylenchlorid, Chloroform, Tetrachlorkohlenstoff, Chlorbenzol und Dichlorbenzole, Ether, wie Diethyl- und Dibutylether, Tetrahydrofuran und Dioxan, Alkohole wie Methanol, Ethanol, n- und iso-Propanol, n-, iso-, sek.- und tert.-Butanol sowie aprotisch polare Lösungsmittel wie Dimethylformamid und Dimethylsulfoxid.

Bei Arbeiten im Zweiphasenmedium werden neben etwa 50 %iger Natronlauge oder Kalilauge organische Lösungsmittel wie Pentan, Hexan, Heptan, Benzol oder Toluol verwendet, welche mit Wasser

nicht mischbar sind.

Als Katalysatoren werden — bei Durchführung des erfindungsgemäßen Verfahrens in mehrphasigen Reaktionsmedien — Verbindungen verwendet, welche gewöhnlich bei Reaktionen in mehrphasigen Systemen als Hilfsmittel zum Phasentransfer von Reaktanden dienen. Insbesondere seien als solche Phasentransfer-Katalysatoren Tetraalkyl- und Trialkyl-benzyl-ammoniumsalze, wie z.B. Tetrabutylammoniumbromid und Trimethylbenzyl-ammonium-chlorid, genannt.

Als Basen können bei der Durchführung des erfindungsgemäßen Verfahrens die bei Carbonyl-Olefinierungen üblichen Basen verwendet werden. Genannt seien Alkalihydroxide wie z.B. Kalium- und Natrium-hydroxid, Alkalialkoholate wie z.B. Kalium- und Natriummethylat, -ethylat, -n- und -iso-propylat, -n-, -iso-, -sek.- und -tert.-butylat, Alkalihydride wie z.B. Natriumhydrid sowie Alkyllithiumverbindungen wie z.B. Butyllithium. Vorzugsweise werden die genannten Alkalihydroxide und/oder -alkoholate verwendet.

Das Verfahren wird im allgemeinen bei Normaldruck durchgeführt. Die Reaktionstemperaturen liegen zwischen − 70 und + 150 °C, vorzugsweise zwischen − 20 und + 50 °C.

Zur Durchführung des erfindungsgemäßen Verfahrens werden die Ausgangsverbindungen der Formeln (II) und (III) gewöhnlich in äquimolaren Mengen eingesetzt. Bei Arbeiten in einphasigen Systemen verwendet man im allgemeinen zwei Basenäquivalente, bei Verwendung von 50 %igen Alkalilaugen als Zweitphasen im allgemeinen das 5 bis 15-fache der stöchiometrisch erforderlichen Menge.

Man legt die Base und gegebenenfalls den Katalysator in einem geeigneten Reaktionsmedium vor und gibt dazu gleichzeitig oder nacheinander — gegebenenfalls in einem der angegebenen Solventien gelöst — die Ausgangsstoffe der Formeln (II) und (III). Zur Vervollständigung der Reaktion wird das Gemisch mehrere Stunden gerührt.

Die Aufarbeitung kann nach üblichen Methoden erfolgen. Zur Isolierung destillierbarer Produkte kann man beispielsweise das Lösungsmittel abdestillieren, den Rückstand in einem mit Wasser nicht mischbaren Lösungsmittel, wie z.B. Ligroin, aufnehmen, die Lösung mit Wasser waschen, trocknen, filtrieren und destillieren. Zur Isolierung kristalliner Produkte kann man gegebenenfalls das Reaktionsgemisch in (Eis)-Wasser gießen und vom kristallinen Produkt absaugen, oder man kann das Reaktionsgemisch — falls das Lösungsmittel nicht mit Wasser mischbar ist — mit Wasser waschen, trocknen, filtrieren und eindampfen. Nach der letztgenannten Aufarbeitungsmethode können auch ölige, schwer destillierbare Produkte relativ rein erhalten werden.

Die Charakterisierung der Produkte erfolgt durch ihre Schmelz- oder Siedepunkte.

Nach dem erfindungsgemäßen Verfahren herzustellende Verbindungen der Formel (I) können als Zwischenprodukte zur Herstellung von Insektiziden verwendet werden (vergleiche DE-OS 2 738 150).

1. Beispiele zu Verfahren (1)

Beispiel 1

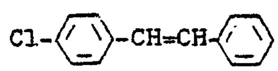

22,8 g (0,1 Mol) Benzylphosphonsäure-diäthylester werden zu einer auf 0 bis 5 °C gekühlten Lösung von 22,4 g (0,2 Mol) Kalium-tert.-butylat in 100 ml Tetrahydrofuran (über Natrium getrocknet) innerhalb von 15 Minuten tropfenweise gegeben. Das Gemisch wird eine Stunde lang gerührt ; dann gibt man dazu unter Kühlen auf 0 bis 5 °C eine Lösung von 28 g (0,1 Mol) α-Hydroxy-4-chlor-benzyl-phosphonsäurediäthylester in 50 ml Tetrahydrofuran und rührt die Mischung noch 15 Stunden bei Raumtemperatur (ca. 20 °C). Dann wird in 1 l Eiswasser gegossen, abgesaugt und getrocknet. Man erhält so 18 g (84 % der Theorie) 1-(4-Chlor-phenyl)-2-phenyl-äthylen in Form farbloser Kristalle vom Schmelzpunkt 130 °C.

Beispiel 2

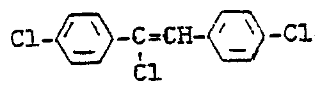

28 g (0,1 mol) α-Hydroxy-4-chlor-benzyl-phosphonsäure-diäthylester und 30 g (0,1 Mol) α-Chlor-4-chlor-benzyl-phosphonsäure-diäthylester werden in 50 ml Toluol gelöst und diese Lösung wird innerhalb von 30 Minuten zu einer intensiv gerührten Mischung aus 100 ml Toluol, 100 ml 40 %iger Natronlauge und 2 g Tetrabutyl-ammonium-bromid bei einer Innentemperatur von 30 bis 35 °C tropfenweise gegeben. Man rührt die Mischung noch 15 Stunden bei Raumtemperatur (ca. 20 °C), trennt die organische Phase ab, wäscht diese zweimal mit Wasser, trocknet, filtriert und destilliert das Lösungsmittel im Vakuum ab. Der

Rückstand wird aus Ligroin kristallin erhalten : 14 g (49 % der Theorie) 1-Chlor-1-(4-chlor-phenyl)-2-(4-chlor-phenyl)-äthylen ; Schmelzpunkt 89 °C.

Beispiel 3

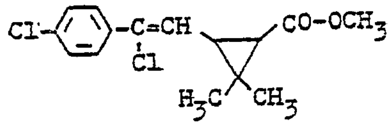

30 g (0,1 Mol) α-Chlor-4-chlor-benzyl-phosphonsäurediäthylester werden zu einer auf 0 bis 10 °C gekühlten Lösung von 8,8 g (0,2 Mol) Natriummethylat in 200 ml Tetrahydrofuran tropfenweise gegeben. Die Mischung wird eine Stunde bei 0 °C gerührt ; dann gibt man dazu bei 0 bis 10 °C eine Lösung von α-Hydroxy-α-(3-methoxy-carbonyl-2,2-dimethyl-cycloprop-1-yl)-methanphosphonsäure-dimethylester in 50 ml Tetrahydrofuran, rührt das Reaktionsgemisch 15 Stunden bei Raumtemperatur (ca. 20 °C), destilliert das Lösungsmittel ab, nimmt den Rückstand in Ligroin auf, wäscht die Lösung mit Eiswasser, trocknet, filtriert und destilliert.

Man erhält 18 g (60 % der Theorie) 3-(2-Chlor-2-(4-chlor-phenyl)-vinyl)-2,2-dimethyl-cyclopropan-1-carbonsäure-methylester vom Siedepunkt 100 °C/0,01 mBar.

Beispiel 4

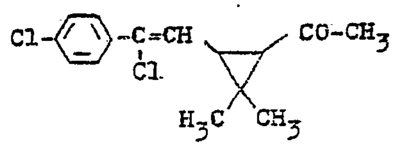

30 g (0,1 Mol) α-Chlor-4-chlor-benzyl-phosphonsäurediäthylester, gelöst in 50 ml Tetrahydrofuran, werden zu einer auf 0 bis 10 °C gekühlten Lösung von 8,8 g (0,2 Mol) Natriummethylat in 200 ml Tetrahydrofuran tropfenweise gegeben. Die Mischung wird eine Stunde gerührt ; dann gibt man dazu bei 0 bis 5 °C eine Lösung von 25 g (0,1 Mol) α-Hydroxy-α-(3-acetyl-2,2-dimethyl-cycloprop-1-yl)-methanphosphonsäure-dimethylester in 100 ml Tetrahydrofuran, rührt das Reaktionsgemisch 15 Stunden bei 15 bis 25 °C, destilliert das Lösungsmittel ab, nimmt den Rückstand in Toluol auf, wäscht dreimal mit Wasser, trocknet, filtriert und destilliert.

Man erhält 18 g (64 % der Theorie) 3-(2-Chlor-2-(4-chlor-phenyl)-vinyl)-2,2-dimethyl-cycloprop-1-yl)-methyl-keton vom Siedepunkt 90 °C/0,01 mBar. Analog erhält man.

Beispiel 5

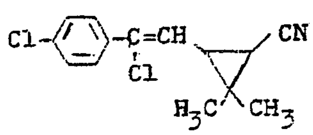

Siedepunkt 94 °C/0,01 mBar

Als Ausgangsstoffe zu verwendende α-Hydroxy-phosphonsäureester der Formel (II) können beispielsweise wie folgt hergestellt werden :

2. Beispiele zu Verfahren (3)

6. 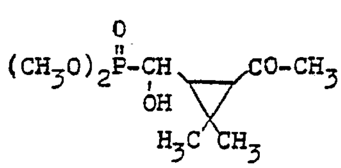

Eine Lösung von 50 g (0,2 Mol) α-oxo-α-(3-acetyl-2,2-dimethyl-cycloprop-1-yl)-methanphosphonsäuredimethylester in 50 ml Methylenchlorid wird zu einer auf 0 bis 5 °C gekühlten Mischung aus 2,5 g

Natriumtetrahydridoborat, 100 ml Wasser und 100 ml Methylenchlorid tropfenweise gegeben und das Reaktionsgemisch wird zwei Stunden bei 0 bis 5 °C gerührt. Dann wird die wässrige Phase von der organischen Phase abgetrennt und noch zweimal mit Methylenchlorid extrahiert. Die vereinigten organischen Phasen werden getrocknet, filtriert und eingedampft. Nach Umkristallisieren des zurückbleibenden Rohproduktes aus 200 ml Essigester/Ligroin (2 : 8) erhält man 31 g (62 % der Theorie) α-Hydroxy-α-(3-acetyl-2,2-dimethyl-cycloprop-1-yl)-methanphosphonsäuredimethylester vom Schmelzpunkt 104 °C.

Analog vorstehendem Beispiel erhält man

7. 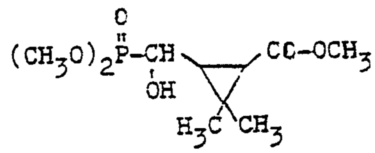 Schmelzpunkt 69 °C

8. 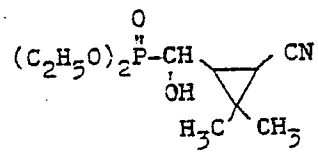

Analog zu vorstehenden Beispiel 6 ist auch nachstehende Verbindung 9 erhältlich, welche der allgemeinen Formel IIIa entspricht :

9. 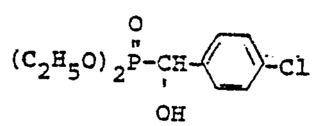

3. Vorschrift zur Herstellung von α-Oxophosphonsäureestern der allgemeinen Formel IV

10. 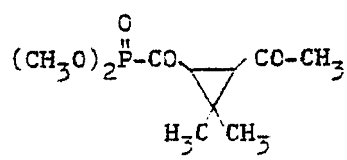

6,5 g (0,05 Mol) Trimethylphosphit werden zu einer auf 35 bis 40 °C erwärmten Lösung von 9 g (0,05 Mol) 3-Acetyl-2,2-dimethyl-cyclopropan-1-carbonsäurechlorid in 20 ml Methylenchlorid tropfenweise gegeben und das Reaktionsgemisch wird 15 Stunden bei 15 bis 25 °C gerührt. Nach Abdestillieren des Lösungsmittels im Vakuum erhält man 9 g (72 % der Theorie) α-Oxo-α-(3-acetyl-2,2-dimethylcycloprop-1-yl)-methan-phosphonsäure-dimethylester.

Analog vorstehender Vorschrift erhält man

11. 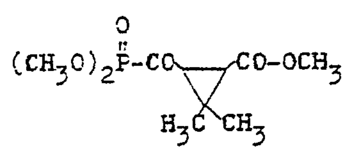

12. 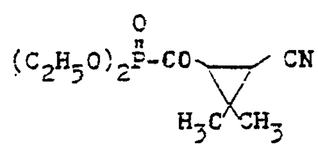

Analog zu vorstehender Vorschrift 10 ist auch die nachstehende Verbindung 13 erhältlich :

13. 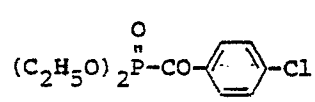

4. Vorschrift zur Herstellung der Carbonsäurechloride (VI)

14. 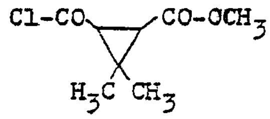

Eine Mischung aus 172 g (1 Mol) 3-Methoxycarbonyl-2,2-dimethyl-cyclopropan-1-carbonsäure, 130 g Thionylchlorid, 2 ml Dimethylformamid, und 200 ml Methylenchlorid wird vier Stunden unter Rückfluß zum Sieden erhitzt. Man erhält nach Destillation im Vakuum 135 g (71 % der Theorie) 3-Methoxycarbonyl-2,2-dimethyl-cyclopropan-1-carbonsäurechlorid vom Siedepunkt 86 °C/15 mBar.

Analog erhält man

15. 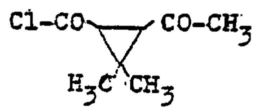

16. 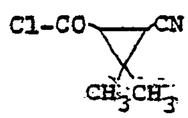 Siedepunkt 95-98 °C/10 mbar

**Ansprüche**

1. Verfahren zur Herstellung von Alkenen der Formel (I)

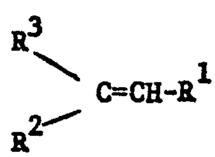 (I)

in welcher

$R^1$ für einen gegebenenfalls substituierten Alkyl-, Alkenyl-, Alkinyl-, Cycloalkyl-, Cycloalkenyl-, Aralkyl-, Aralkenyl- oder Aryl-Rest oder für einen gegebenenfalls substituierten heterocyclischen Rest steht,

$R^2$ für Wasserstoff oder Halogen steht und

$R^3$ für Halogen, Cyano, für einen gegebenenfalls substituierten Alkyl-, Alkenyl-, Alkinyl-, Cycloalkyl-, Aralkenyl-, Aryl-, Alkylcarbonyl-, Arylcarbonyl-, Alkoxycarbonyl- oder Aminocarbonyl-Rest oder für einen gegebenenfalls substituierten heterocyclischen Rest steht,

dadurch gekennzeichnet, daß man α-Hydroxy-phosphonsäureester der Formel II

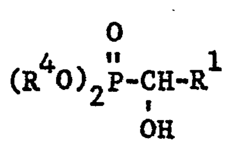 (II)

in welcher

$R^1$ die oben angegebene Bedeutung hat und

$R^4$ für Alkyl oder phenyl steht oder die beiden Reste $R^4$ zusammen für geradkettiges oder verzweigtes Alkandiyl (Alkylen) stehen,

mit phosphorhaltigen Olefinierungsmitteln der Formel III

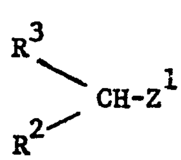 (III)

in welcher

$R^2$ und $R^3$ die oben angegebenen Bedeutungen haben und

$Z^1$ für einen phosphorhaltigen Rest

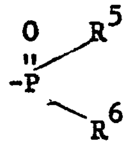 oder $-P(R^7)_3^{\oplus}$ $X^{\ominus}$

steht, wobei

$R^5$ und $R^6$ gleich oder verschieden sind und einzeln für Alkyl, Phenyl, Alkoxy oder Phenoxy oder zusammen für Alkylendioxy stehen,

$R^7$ für Alkyl oder Phenyl steht und

X für Halogen steht,

in Gegenwart von Basen, gegebenenfalls in Gegenwart von Phasentransferkatalysatoren, und gegebenenfalls unter Verwendung von Verdünnungsmitteln bei Temperaturen zwischen − 70 und + 150 °C umsetzt.

2. α-Hydroxy-phosphonsäureester der Formel IIa

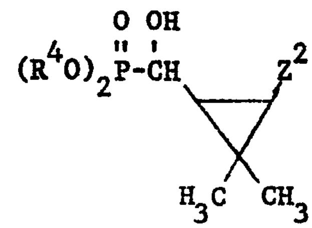

(IIa)

in welcher

$R^4$ für Alkyl oder Phenyl steht oder die beiden Reste $R^4$ zusammen für geradkettiges oder verzweigtes Alkandiyl (Alkylen) stehen und

$Z^2$ für Cyano, Carbamoyl, Acetyl oder $C_1$-$C_4$-Alkoxycarbonyl steht.

3. Verfahren zur Herstellung von α-Hydroxyphosphonsäureestern der im Anspruch 2 angegebenen Formel IIa

dadurch gekennzeichnet, daß man α-Oxo-phosphonsäureester der Formel IV

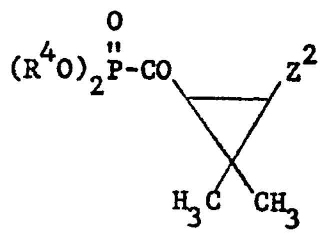

(IV)

in welcher

$R^4$ und $Z^2$ die im Anspruch 2 angegebenen Bedeutungen haben,

mit Hydridkomplexen der Formel V

$$M(M'H_4) \quad (V)$$

in welcher

M für Lithium, Natrium oder Kalium steht und

M′ für Bor oder Aluminium steht,

gegebenenfalls in Gegenwart eines Puffermittels und gegebenenfalls unter Verwendung eines Verdünnungsmittels bei Temperaturen zwischen − 20 und + 50 °C umsetzt.

**Claims**

1. Process for the preparation of alkenes of the formula (I)

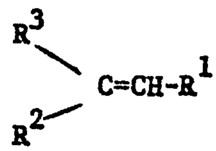

(I)

in which

$R^1$ represents an optionally substituted alkyl, alkenyl, alkinyl, cycloalkyl, cycloalkenyl, aralkyl, aralkenyl or aryl radical or an optionally substituted heterocyclic radical,

$R^2$ represents hydrogen or halogen and

$R^3$ represents halogen, cyano, an optionally substituted alkyl, alkenyl, alkinyl, cycloalkyl, aralkenyl, aryl, alkylcarbonyl, arylcarbonyl, alkoxycarbonyl or amino-carbonyl radical or an optionally substituted heterocyclic radical,

characterised in that α-hydroxy-phosphonic acid esters of the formula II

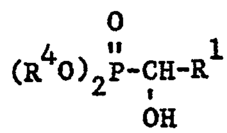

(II)

in which

$R^1$ has the meaning indicated above and

$R^4$ represents alkyl or phenyl or the two radicals $R^4$ together represent straight-chain or branched alkanediyl (alkylene),

are reacted with phosphorus-containing olefinating agents of the formula III

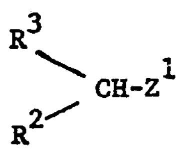

(III)

in which

$R^2$ and $R^3$ have the meanings indicated and

$Z^1$ represents a phosphorus-containing radical

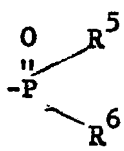

or

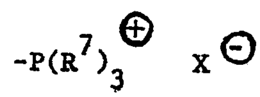

wherein

$R^5$ and $R^6$ are identical or different and individually represent alkyl, phenyl, alkoxy or phenoxy or together represent alkylenedioxy,

$R^7$ represents alkyl or phenyl and

X represents halogen,

in the presence of bases, if appropriate in the presence of phase transfer catalysts, and if appropriate using diluents, at temperatures between − 70 and + 150 °C.

2. α-Hydroxy-phosphonic acid esters of the formula IIa

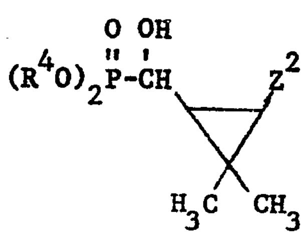

(IIa)

in which

$R^4$ represents alkyl or phenyl or the two radicals $R^4$ together represent straight-chain or branched

alkanediyl (alkylene) and

$Z^2$ represents cyano, carbamoyl, acetyl or $C_1$-$C_4$-alkoxy-carbonyl.

3. Process for the preparation of α-hydroxyphosphonic acid esters of the formula IIa given in Claim 2, characterised in that α-oxo-phosphonic acid esters of the formula IV

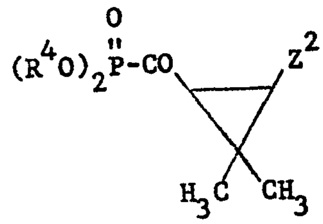

(IV)

in which

$R^4$ and $Z^2$ have the meanings indicated in Claim 2, are reacted with hydride complexes of the formula V

$$M(M'H_4) \qquad (V)$$

in which

M represents lithium, sodium or potassium and

M' represents boron or aluminium,

if appropriate in the presence of a buffer and if appropriate using a diluent, at temperatures between − 20 and + 50 °C.

**Revendications**

1. Procédé pour la préparation d'alcènes de formule I

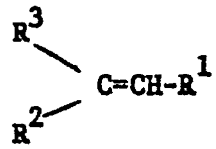

(I)

dans laquelle

$R^1$ représente un reste alkyle, alcényle, alcynyle, cycloalkyle, cycloalcényle, arylalkyle, arylalcényle ou aryle éventuellement substitué ou un reste hétérocyclique éventuellement substitué,

$R^2$ représente l'hydrogène ou un halogène et

$R^3$ représente un halogène ou un groupe cyano, un reste alkyle, alcényle, alcynyle, cycloalkyle, arylalcényle, aryle, alkylcarbonyle, arylcarbonyle, alcoxycarbonyle ou aminocarbonyle éventuellement substitué ou un reste hétérocyclique éventuellement substitué,

caractérisé en ce que l'on fait réagir des esters d'acides α-hydroxyphosphoniques de formule II

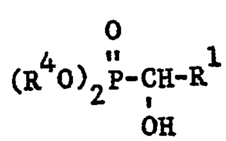

(II)

dans laquelle

$R^1$ a la signification indiquée ci-dessus et

$R^4$ représente un reste alkyle ou phényle, ou bien les deux restes $R^4$ représentent ensemble un reste alcanediyle (alkylène) à chaîne droite ou ramifiée,

à des températures comprises entre − 70 et + 150 °C avec des agents d'oléfinisation phosphorés de formule III

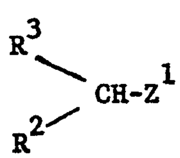

(III)

dans laquelle

$R^2$ et $R^3$ ont les significations indiquées ci-dessus et

$Z^1$ représente un reste phosphoré

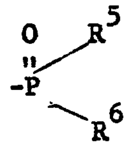

ou $-P(R^7)_3^{\oplus}$ $X^{\ominus}$

dans lesquels

$R^5$ et $R^6$ sont identiques ou différents et représentent chacun un reste alkyle, phényle, alcoxy ou phénoxy ou bien représentent ensemble un reste alkylènedioxy,

$R^7$ représente un reste alkyle ou phényle et

X représente un halogène,

en présence de bases, éventuellement en présence de catalyseurs de transfert de phases et éventuellement avec utilisation d'agents diluants.

2. Esters d'acides α-hydroxyphosphoniques de formule IIa

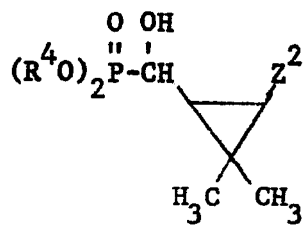

(IIa)

dans laquelle

$R^4$ représente un reste alkyle ou phényle, ou bien les deux restes $R^4$ pris ensemble représentent un reste alcanediyle (alkylène) à chaîne droite ou ramifiée, et

$Z^2$ représente un groupe cyano, carbamoyle, acétyle ou (alcoxy en $C_1$-$C_4$)-carbonyle.

3. Procédé pour la préparation d'esters d'acides α-hydroxyphosphoniques de formule IIa indiquée dans la revendication 2, caractérisé en ce que l'on fait réagir des esters d'acides α-oxophosphoniques de formule IV

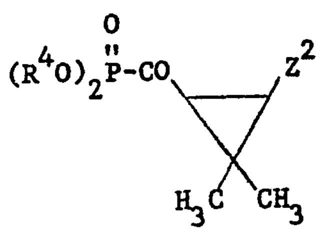

(IV)

dans laquelle

$R^4$ et $Z^2$ ont les significations indiquées dans la revendication 2 à des températures comprises entre − 20 et + 50 °C avec des hydrures complexes de formule V

$$M(M'H_4) \quad (V)$$

dans laquelle

M représente le lithium, le sodium ou le potassium et

M' représente le bore ou l'aluminium,

éventuellement en présence d'un agent tampon et éventuellement avec utilisation d'un agent diluant.